# EUROPEAN PATENT SPECIFICATION

(11) **EP 1 047 671 B1**
(45) Date of publication and mention of the grant of the patent: **10.12.2003**
(21) Application number: 98960122.4
(22) Date of filing: 29.12.1998
(51) Int. Cl.: C07C 401/00

(54) **METHOD OF PREPARATION OF CHOLECALCIFEROL DERIVATIVES AND NEW INTERMEDIATE COMPOUNDS**
VERFAHREN ZUR HERSTELLUNG VON CHOLECALCIFEROL-DERIVATEN UND ZWISCHENVERBINDUNGEN
PROCEDE DE PREPARATION DE DERIVES DE CHOLECALCIFEROL ET NOUVEAUX COMPOSES INTERMEDIAIRES

(30) Priority: 14.01.1998 PL 32427498
(43) Date of publication of application: 02.11.2000
(73) Proprietor: INSTYTUT FARMACEUTYCZNY, PL-01-793 Warszawa (PL)
(72) Inventor: KUTNER, Andrzej, PL-02-785 Warszawa (PL); CHODYNSKI, Micha$m(D), PL-05-800 Pruszków (PL); SZELEJEWSKI, Wies$m(D)aw, PL-03-977 Warszawa (PL); ODRZYWOLSKA, Ma$m(D)gorzata, PL-22-400 Zamosc (PL); FITAK, Hanna, PL-01-840 Warszawa (PL)
(74) Representative: König, Beate, Dipl.-Phys. Dr.
(86) International application number: PL9800051
(87) International publication number: WO99036400

(56) References cited:
- EP-A- 0 230 600
- EP-A- 0 377 743
- R.M. MORIARTY ET AL: TETRAHEDRON LETT., vol. 36, no. 29, 1995, pages 5139-5142, XP004027625
- P. KOCH ET AL: BULL. SOC. CHIM. FR. II, no. 7/8, 1983, pages 189-194, XP002101156

## Description

The object of the invention is a new method of preparation of cholecalciferol derivatives, particularly 1α,24-dihydroxy-cholecalciferol, and new intermediate compounds used in the method.

Cholecalciferol derivatives, that are active metabolites of vitamin D₃, exhibit activity that regulates calcium homeostasis in the body as well as initiate cell differentiation. This property, exhibited particularly by 1α,24(R)-dihydroxycholecalciferol, make it possible to use the said derivatives in treatment of a serious skin disease, psoriasis, caused by incomplete differentiation and rapid proliferation of epidermal cells.

The known method of preparation of cholecalciferol derivatives, such as for example 1α,24(R)-dihydroxycholecalciferol (known as tacalcitol), and its stereomer of 1α,24(S) configuration, described in the Dutch Patent Application No. 7507268, consists in subjecting natural, hard available fucosterol to multistep linear synthesis. The main step of the synthesis includes reaction of ozonization of the double bond in the side chain of fucosterol and reduction of the resulting carbonyl group to the hydroxyl group. After more than ten steps, mixture of diastereomeric alcohols is obtained which has to be separated by chromatography on silica gel impregnated with AgNO₃.

Other methods, disclosed for example in Japanese patent publications No.J5 1076253 and J5 2042864, include methods of modification of steroid precursor backbone of cholestadiene, containing a carbon side chain substituted with hydroxyl at C-24.

All these methods are time- and labour-consuming because of multistep synthesis and chromatographic separation of the resulting diastereomer mixtures.

A method of stereoselective total synthesis of 1α,24(R)-dihydroxycholecalciferol starting from fragments of A ring and CD ring system of vitamin D molecule and involving intermediate dienyl derivative is also described in the literature (Tetrahedron, 53, p.4703 (1997)). The key step of side chain synthesis is an opening of a chiral epoxy compound with a use of nitrile anion of Inhoffen-Lythgoc diol precursor.

The objective of the present invention was to develop a practical and effective method of synthesis of cholecalciferol derivatives starting from readily available raw materials, that would enable preparation of optically pure cholecalciferol derivatives directly from chemical synthesis without a subsequent step of diastereomer mixture separation.

The essential feature of the present invention is a method of preparation of cholecalciferol derivatives, particularly 1α,24-dihydroxycholecalciferol, of general formula protected 1, wherein R₁ and R₂ are hydrogen, hydroxyl or protected hydroxyl group, R₁ and R₂ being the same or different, that consists in reacting phenylsulfonyl derivative of formula 2, wherein R₁ and R₂ are hydrogen or protected hydroxyl group, with 1,2-epoxy-3-methylobutane of (S) or (R) configuration, respectively, at carbon atom C-2 prepared in a separate process or "in situ". The reaction is carried out in the presence of an aprotic solvent and a strong organic base, to yield hydroxysulfon of (R) or (S) configuration, respectively, of formula 3a, wherein R₁ and R₂ are as defined above, and the configuration at carbon atom C-22 is represented by formula 3c and 3d. In the next step, a phenylsulfonyl group is removed from hydroxysulfone of formula 3a by means of reduction, to yield a compound of formula 4, wherein the carbon atom C-24 is of configuration represented by formula 4a or 4b, from which optionally hydroxyl protecting groups are removed using a known method.

As organometallic compounds, lithium, sodium or potassium organic compounds are used, preferably butyl lithium.

As aprotic solvents, alkylated phosphoric acid amides or urea alkyl derivatives, preferably hexamethyltriamide of phosphoric acid (HMPT), are used.

Phenylsulfonyl group may be removed by reduction with metals, preferably with sodium amalgamate.

Hydroxyl protecting group is any protecting group used in vitamin D chemistry to protect hydroxyl groups, such as for example acyl, alkylsilyl or alkoxyalkyl group.

The term "acyl group" includes alkanoyl and carboxyal-kanoyl groups, of 1 to 6 carbon atoms, such as in particular acetate group. Alkylsilyl protecting groups are such groups as trimethylsilyl, triethylsilyl, t-butyldimethylsilyl. Typical alkoxyalkyl groups are: metoxymethyl, etoxymethyl, tetrahydrofuranyl and tetrahydropyranyl.

The object of the invention are also new hydroxysulfones represented by the formulae 3a and 3b, prepared as stereomers of different configuration at carbon atoms C-22 and C-24. In these formulae substituents R₁ and R₂ at carbon atoms C-1 and C-3 denote hydrogen or protected hydroxyl group, R₁ and R₂ being the same or different.

A further aspect of the invention are new compounds of formula 1, of configuration at carbon atom C-24 represented by formulae 4a or 4b, wherein R₁ and R₂ denote hydrogen or protected hydroxyl group, R₁ and R₂ being the same or different.

All the new compounds are intermediate compounds in the method of the invention.

The starting compounds for cholecalciferol derivatives synthesis: C-22 vitamin sulfones represented by formula 2, are key synthons used in chemistry of vitamins.

Sulfones of formula 2 may be prepared from the respective C-22 alcohols using known methods, analogously to preparation of C-25 sulfones with C-25 vitamin alcohols, as described in Polish patent No. 179799.

The method according to the invention enables to prepare cholecalciferol derivatives, particularly substituted with hydroxyl group at position 24, such as for example 1α,24(R)-dihydroxycholecalciferol of formula 1a and 1α,24(S)-dihydroxycholecalciferol of formula 1b, starting from compounds readily commercially available or synthesised in a simple way.

On the Scheme 1 preparation of 1α,24(R)-dihydroxycholecalciferol by the method according to the invention is presented.

On the Scheme 2 preparation of 1α,24(S)-dihydroxycholecalciferol by the method according to the invention is presented.
The invention is illustrated by the following examples, which do not limit the scope of the invention.

### Example 1.

Preparation of (5Z,7E)-(1S,3R,24R)-1,3-di-tert-butyldimethylsilyl-22-phenylsulfonyl-9,10-secocholesta-5,7,10(19)-triene-24-ol (formulae 3c and 3d)
Solution of sulfone of formula 2 wherein R₁ = R₂ = tert-butyldimethylsilyloxy (TBSO), (110 mg, 158 µmol) in 800 µl THF, containing several crystals of 1,10-phenanthroline, was cooled to -70°C and then solution of n-BuLi in hexane (150 µL, 1.6 M, 240 µmole) was added. The solution was stirred at -70°C for 20 min and (S)-1,2-epoxy-3-methylbutane {[α]²⁵_{D} = +4.3° c 2 MeOH, 50 µl, 580 µmole} diluted with 50 µl THF was added. The solution was stirred for 20 min at -70°C and then 100 ml HMPT was added. The stirring was continued at -30°C for 5 h, and then the solution was allowed to stand at -18°C for 15 h until the substrate disappeared (TLC using 18% ethyl acetate in hexane system). To the mixture 0.5 ml brine was added and the products were extracted with ethyl acetate. The products were dissolved in benzene and subjected to "flash" column chromatography on silica gel (300 - 400 mesh). The hydroxysulfones of formulae 3c and 3d (total amount: 75 mg, 60%) were isolated using solvent mixture (hexane - ethyl acetate (8-14%) as colourless oil: UV (EtOH) λ¹ₘₐₓ 265.2 nm; λ²ₘₐₓ 265.0 nm; TLC (18% ethyl acetate in hexane): R¹_{f} = 0.37, R²_{f} = 0.32.

### Example 2.

### Preparation of (5Z,7E)-(1S,3R,24R)-9,10-secocholesta-5,7,10(19)-triene-1,3,24-triol (formula 1a)

To the solution of mixture of sulfones of formulae 3c and 3d (75 mg) in 100 µl THF, 1 ml of methanol saturated with Na₂HPO₄ and solid Na₂HPO₄ were added. The resulting suspension was stirred for 15 min at 20°C. Then, the freshly prepared sodium amalgamate (5% Na/Hg) was added and the suspension was stirred at 40°C for 1 h until the substrate disappeared (TLC using 18% ethyl acetate in hexane system). To the suspension 1 ml ethyl acetate was added and filtered through 5 g silica gel (5 g) using 50 ml mixture eluting 12% ethyl acetate in hexane. Alcohol of formula 4a (52 mg, 84%) was obtained as colourless oil. The oil was dissolved in 2 ml of metanol-chloroform 1:1 mixture, then 15 mg of 10-camphorsulfonic acid was added and the resulting mixture was stirred at 20°C for 2.5 h. The solvents were removed under reduced pressure and the product dissolved in chloroform was subjected to "flash" column chromatography on silica gel (300-400 mesh) (5 g). Chromatographically pure triol of formula 1a (21 mg, 65%) was isolated using a solvent mixture (30-35% ethyl acetate in hexane) as colourless oil. TLC R_{f} value was identical as that of 1,25-dihydroxycholecalciferol in straight phases (75% ethyl acetate in hexane) and reverse phases (8% water in methanol); HPLC, Chiralpak AD column, 4,6 x 25 mm, 15% EtOH in hexane, R_{V} = 9.7 ml; IR (film) 3357, 2946, 1645, 1055 cm⁻¹; UV (EtOH) λₘₐₓ 265.2 nm, λₘᵢₙ 228.6 nm, A₂₆₅/A₂₂₈ = 1.70; [α]_{D}²³ +33.8° (c 0.86 CHCl₃); ¹H NMR (CDCl₃) δ (ppm): 0.55 (3H, s, 18-CH₃), 0.91 and 0.92 (3H and 3H, dd, J=6.8 Hz, 26- and 27-CH₃), 0.94 (3H, d, J=5.7 Hz, 21-CH₃), 3.32 (1H, m, 24-H), 4.23 (m, 1H, 3-H), 4.43 (1H, m, 1-H), 5.00 (1H, d, J=3 Hz, 19Z-H), 5.33 (1H, d, J=3 Hz, 19E-H) , 6.01 (1H, d, J=11.5 Hz, 7-H), 6.38 (1H, d, J=11.5 Hz, 6-H); EIMS m/z (relative intensity) 416 (M⁺, 18), 398 (27), 380 (13), 365 (5), 251 (20), 152 (42), 134 (100), HRMS, calculated for C₂₇H₄₄O 416.3290, found 416.3291.

### Example 3.

### Preparation of (5Z,7E)-(1S,3R,24S)-1,3-di-tert-butyldimethylsilyl-22-phenylsulfonyl-9,10-secocholesta-5,7,10(19)-triene-24-ol (formula 3b)

Hydroxysulfone of formula 3b was prepared from sulfone of formula 2 wherein R₁ = R₂ = tert-butyldimethylsilyloxy (TBSO) (110 mg, 0,158 mmole) and (R)-1,2-epoxy-3-methylbutane {[α]²⁵_{D} = -4.3° c 2.1 MeOH, 40 µl, 0.46 mmole} according to the method described in Example 1. Sulfone of formula 2 (40 mg) was recovered by column chromatography and hydroxysulfone of formula 3b was obtained (70 mg, Yield 89% recalculated to the reacted substrate); TLC, 18% ethyl acetate in hexane, R_{f} =0.34; UV (EtOH) λₘₐₓ =265 nm.

### Example 4.

### Preparation of (5Z,7E)-(1S,3R,24S)-9,10-secocholesta-5,7,10(19)-triene-1,3,24-triol (formula 1b)

Alcohol of formula 4b was prepared from hydroxysulfone of formula 3b (70 mg) according to method described in Example 2. After performing column chromatography, alcohol of formula 4b was obtained (48 mg, Yield 83%) as colourless oil. Triol of formula 1b was obtained from alcohol 4b (48 mg) according to the method described in Example 2. After performing column chromatography, triol 1b (21 mg, Yield 68%) was obtained as colourless oil. Preparative HPLC chromatography (column 22 x 25 cm, Lichrosorb Si 60, 10 mm, 15% propanol-2 in hexane) yielded triol of formula 1b as colourless oil (19 mg): HPLC, column 4.4 x 25 cm, Hibar Si 60, 14% propanol-2 in hexane, R_{V} 12 ml, Chiralpak AD column, 4.6 x 25 cm, 15% EtOH in hexane, R_{V} = 6.6 ml: IR (film) 3371, 2949, 1650, 1063 cm⁻¹; UV (EtOH) λₘₐₓ 265.2 nm, λₘᵢₙ 228.6 nm, A₂₆₅/A₂₂₈ = 1 .73; [α]_{D}²⁴ +23.5° (c 1.07 CHCl₃); ¹H NMR (CDCl₃) δ 0.54 (3H, s, 18-CH₃), 0.90 and 0.93 (3H and 3H, dd, J=6.5 Hz, 26- and 27-CH₃), 0.94 (3H, d, J=5.7 Hz, 21-CH₃), 3.32 (1H, m, 24-H), 4.22 (m, 1H, 3-H), 4.43 (1H, m,1-H), 5.02 (1H, d, J=3 Hz, 19Z-H), 5.32 (1H, d, J=3 Hz, 19E-H), 6.01 (1H, d, J=11.5 Hz, 7-H), 6.39 (1H, d, J=11.5 Hz, 6-H), MS, m/z (relative intensity) 416 (M⁺, 13), 398 (34), 380 (18), 287 (6), 269 (9), 251 (13), 152 (35), 134 (100) ; HRMS calculated for C₂₇H₄₄O₃: 416.3290, obtained: 416.3291.

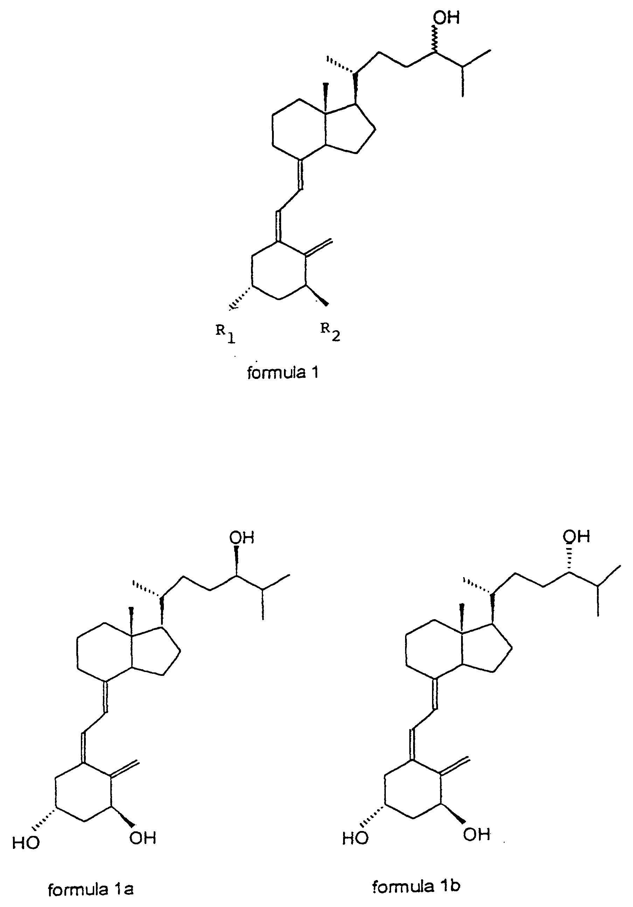

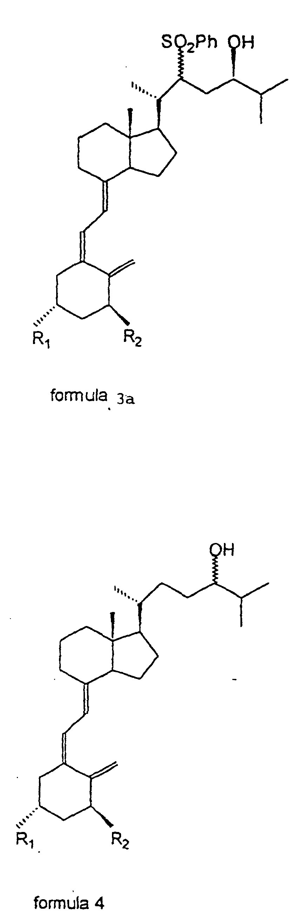

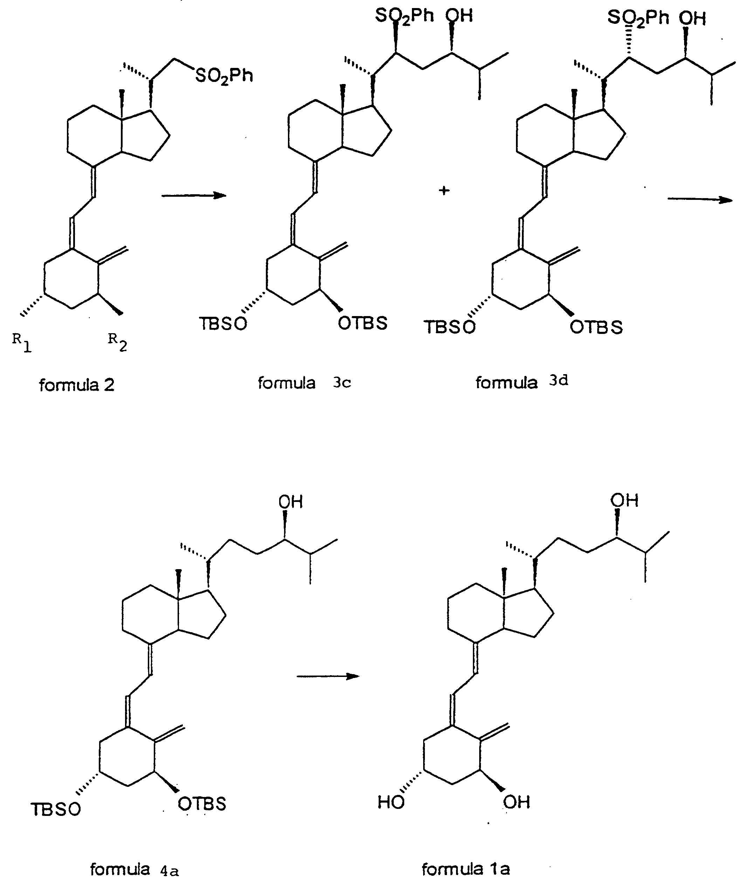

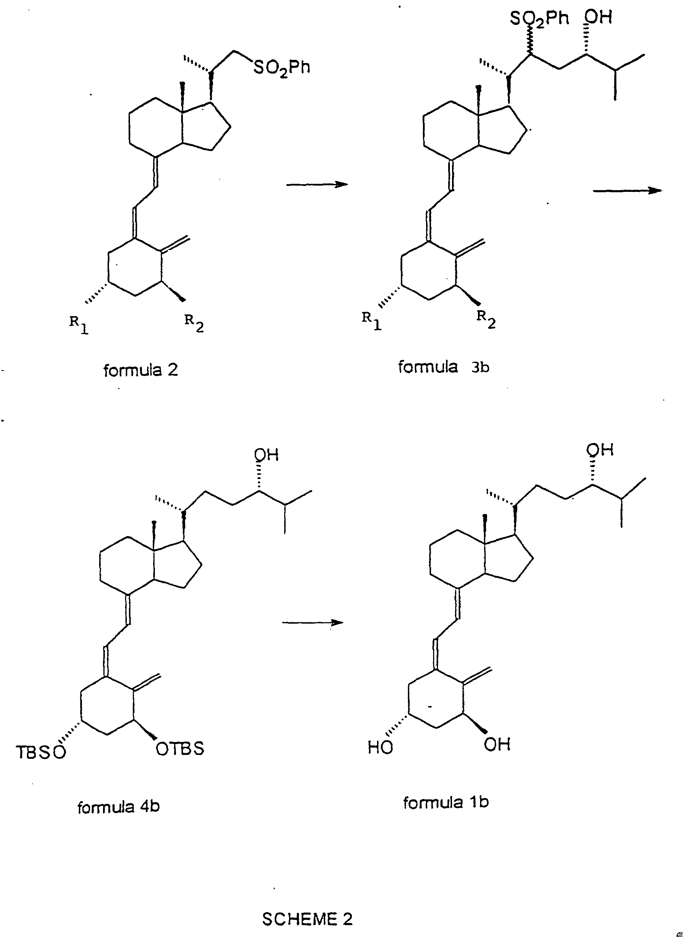

## Claims

1. Method of preparation of cholecalciferol derivatives, particularly 1α,24-dihydroxycholecalciferol, of formula 1, 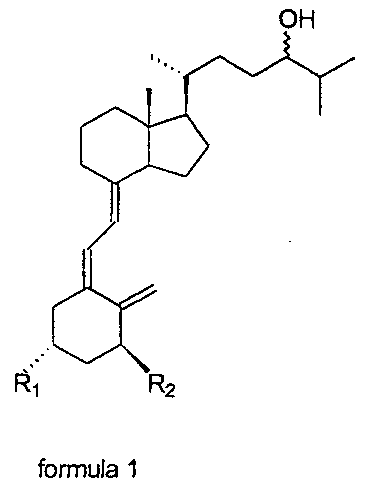 wherein substituents R₁ and R₂ are hydrogen, hydroxyl, or hydroxyl protecting group, R₁ and R₂ being the same or different, wherein phenylsulfonyl derivative of formula 2, 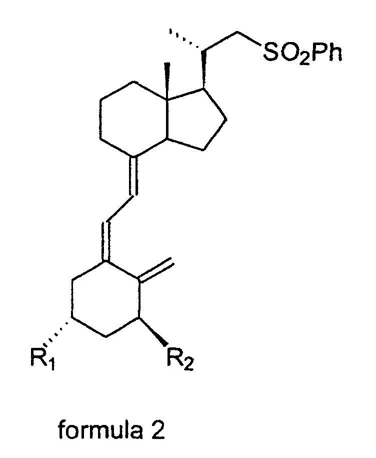 wherein R₁ and R₂ denote hydrogen or hydroxyl protecting group, R₁ and R₂ being the same or different, is reacted with 1,2-epoxy-3-methylbutane of (S) or (R) configuration at carbon atom C-2, respectively, in the presence of an aprotic solvent and a strong organic base, and then from the resulting hydroxysulfone of (R) or (S) configuration, respectively, represented by formula 3a or 3b, 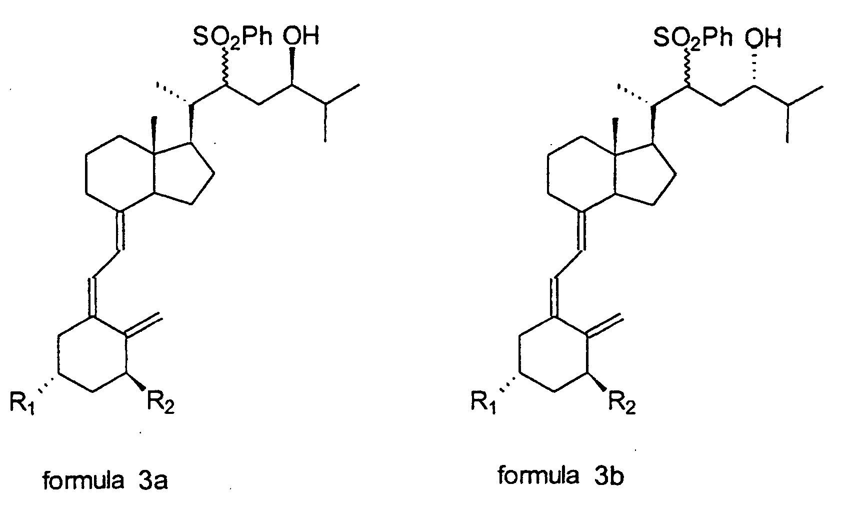 wherein R₁ and R₂ are as defined above in formula 2 above, phenylsulfonyl group is removed by reduction to yield a compound of formula 1, from which optionally hydroxyl protecting groups are removed using the known method.

2. Method according to claim 1, wherein as an organic base lithium organic compounds are used.

3. Method according to claim 1 or 2, wherein as an aprotic solvent hexamethyltriamide of phosphoric acid (HMPT) is used.

4. Method according to claim 1 or 2 or 3, wherein as a reducing compound sodium amalgamate is used.

5. A cholecalciferol derivative, which is a compound of formula 3a, 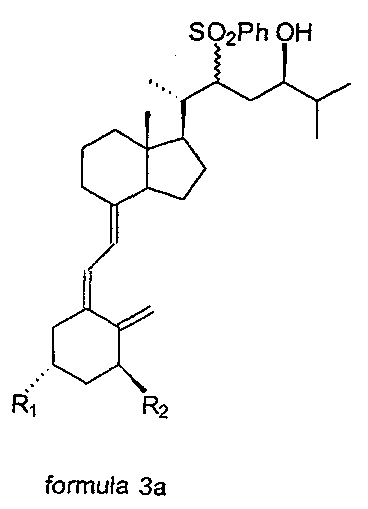 of configuration represented by formulae 3c or 3d, 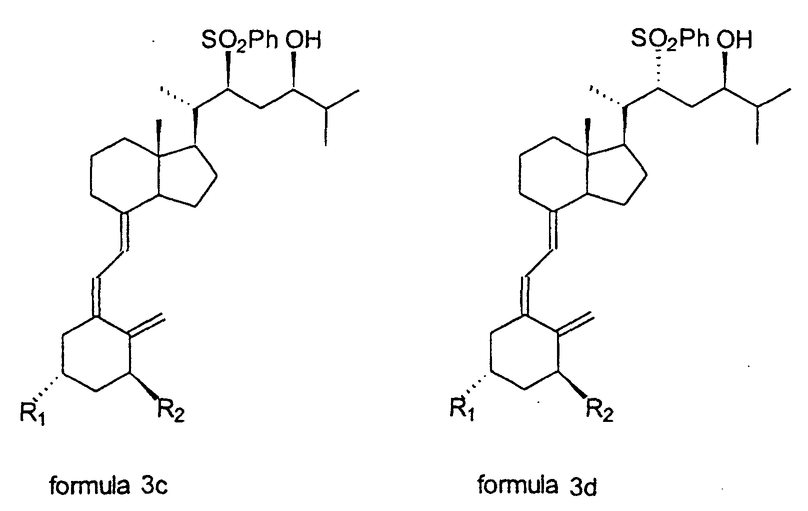 wherein substituents R₁ and R₂ denote hydrogen or hydroxyl protecting group, R₁ and R₂ being the same or different.

6. A cholecalciferol derivative, which is a compound of formula 3b, 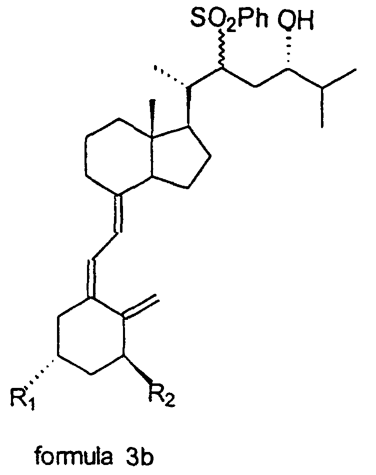 wherein substituents R₁ and R₂ denote hydrogen or protected hydroxyl group, R₁ and R₂ being the same or different.

7. A cholecalciferol derivative, which is a compound of formula 1, 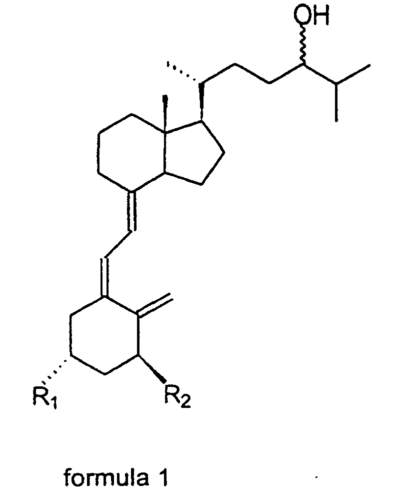 of configuration represented by formulae 4a or 4b, 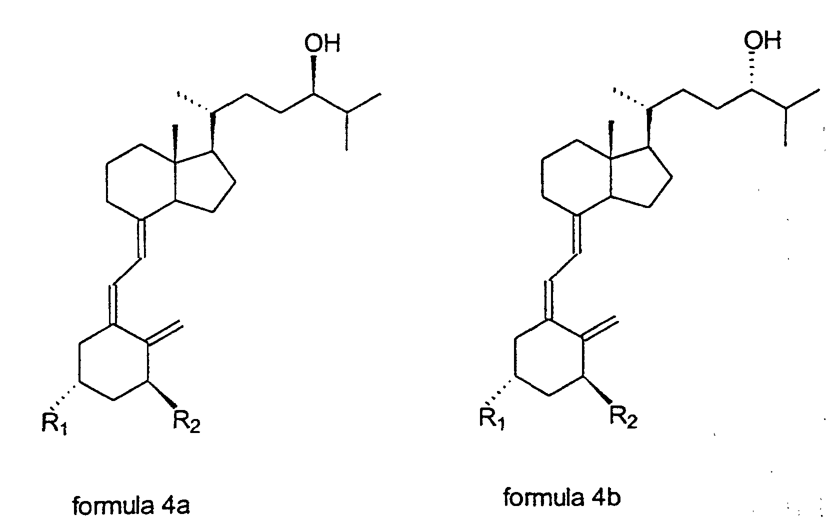 wherein substituents R₁ and R₂ denote hydrogen or hydroxyl protecting group, R₁ and R₂ being the same or different.

## Patentansprüche

1. Verfahren zur Herstellung von Cholecalciferolderivaten, insbesondere von 1α,24-Dihydroxycholecalciferol, der Formel 1 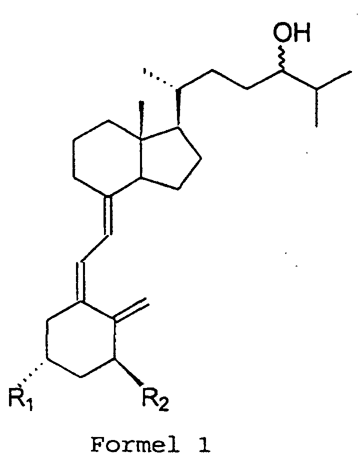 worin die Substituenten R₁ und R₂, die gleich oder verschieden sein können, Wasserstoff, eine Hydroxylgruppe oder eine Hydroxylschutzgruppe bedeuten, wobei ein Phenylsulfonylderivat der Formel 2, 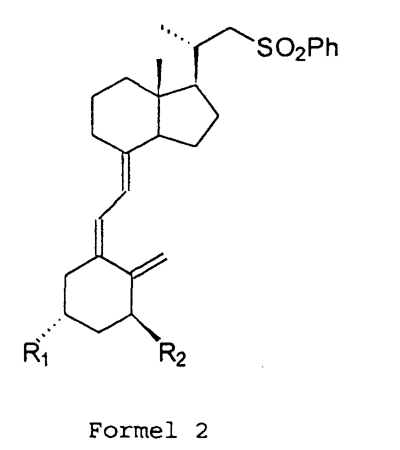 worin R₁ und R₂, die gleich oder verschieden sein können, Wasserstoff oder eine Hydroxylschutzgruppe bedeuten, mit 1,2-Epoxy-3-methylbutan von (S)- oder (R)-Konfiguration am Kohlenstoffatom C-2 in Gegenwart eines aprotischen Lösungsmittels und einer starken organischen Basis umgesetzt und danach aus dem erhaltenen Hydroxysulfon von (R)- bzw. (S)-Konfiguration der Formel 3a oder 3b, 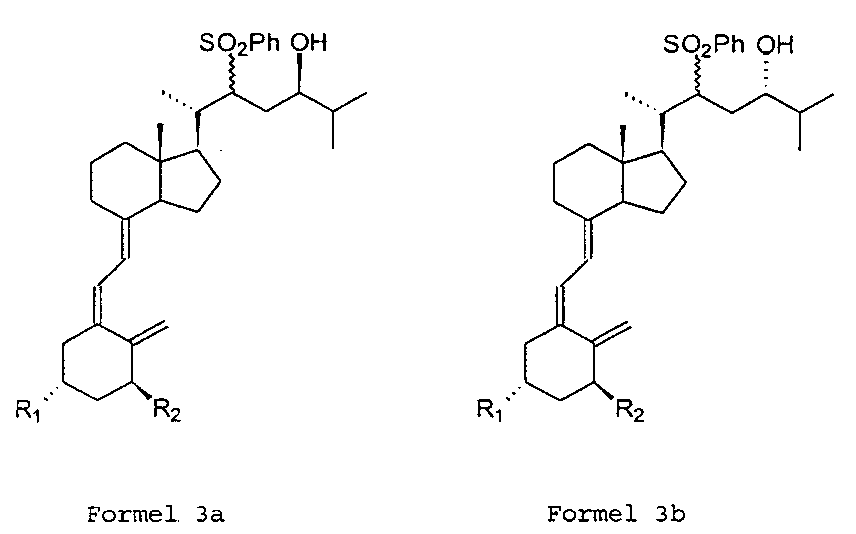 worin R₁ und R₂ die in Formel 2 angegebene Bedeutung besitzen, die Phenylsulfonylgruppe durch Reduktion entfernt und damit eine Verbindung der Formel 1 erhalten wird, von der gewünschtenfalls die Hydroxylschutzgruppen nach bekannten Verfahren entfernt werden.

2. Verfahren gemäß Anspruch 1, wobei als organische Base lithiumorganische Verbindungen verwendet werden.

3. Verfahren gemäß Anspruch 1 oder 2, wobei als aprotisches Lösungsmittel das Hexamethyltriamid von Phosphorsäure (HMPT) verwendet wird.

4. Verfahren gemäß Anspruch 1 oder 2 oder 3, wobei als reduzierende Verbindung Natriumamalgam verwendet wird.

5. Cholecalciferolderivat der Formel 3a 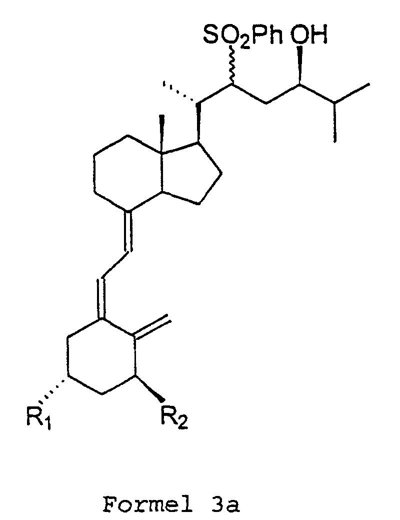 mit der durch Formel 3c oder 3d wiedergegebenen Konfiguration 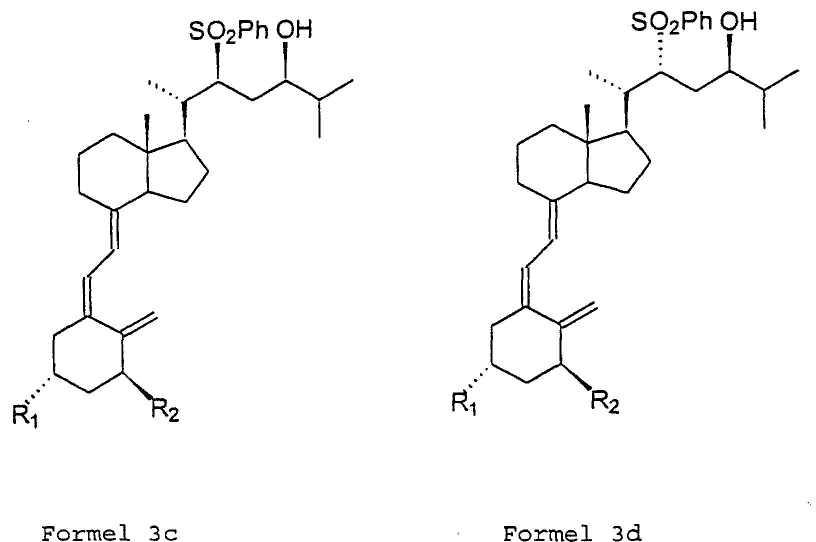 wobei die Substituenten R₁ und R₂, die gleich oder verschieden sein können, Wasserstoff oder eine Hydroxylschutzgruppe bedeuten.

6. Cholecalciferolderivat der Formel 3b 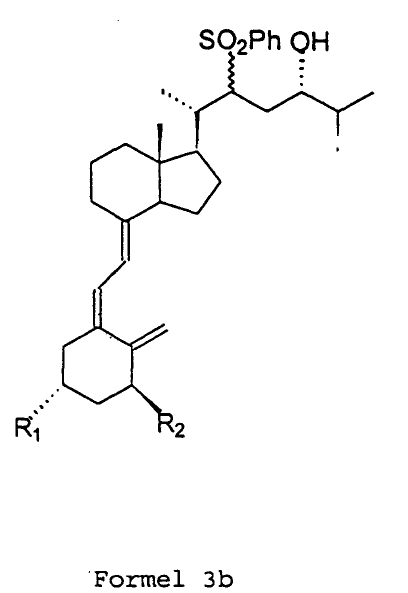 wobei die Substituenten R₁ und R₂, die gleich oder verschieden sein können, Wasserstoff oder eine geschützte Hydroxylgruppe bedeuten.

7. Cholecalciferolderivat der Formel 1 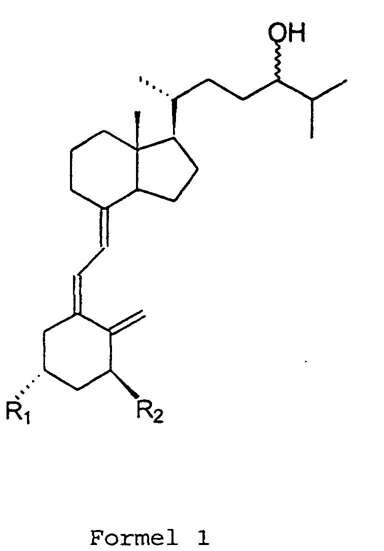 mit der durch die Formeln 4a oder 4b wiedergegebenen Konfiguration, 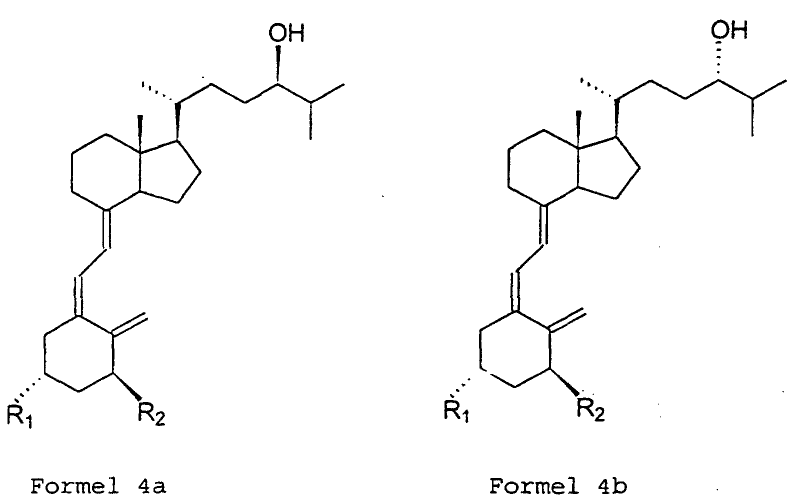 worin R₁ und R₂, die gleich oder verschieden sein können, Wasserstoff oder eine Hydroxylschutzgruppe bedeuten.

## Revendications

1. Procédé de préparation de dérivés de cholécalciférol, en particulier du 1α,24-dihydroxycholécalciférol, de formule 1 : 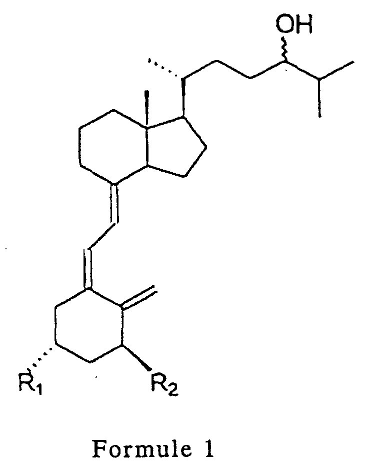 dans laquelle R₁ et R₂ sont identiques ou différents et représentent chacun un atome d'hydrogène, un groupe hydroxyle ou un groupe hydroxy-protecteur,
dans lequel procédé l'on fait réagir un dérivé phénylsulfonylé de formule 2 : 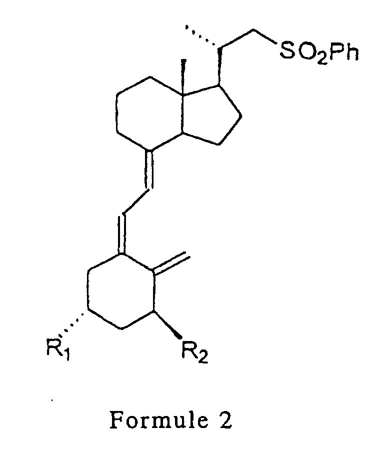 dans laquelle R₁ et R₂ sont identiques ou différents et représentent chacun un atome d'hydrogène ou un groupe hydroxy-protecteur,
avec du 1,2-époxy-3-méthylbutane de configuration (S) ou (R) au niveau de l'atome de carbone n° 2, en présence d'un solvant aprotique et d'une base organique forte, puis on élimine par réduction le groupe phénylsulfonyle de l'hydroxysulfone résultante de configuration respective (R) ou (S), représentée respectivement par la formule 3a ou 3b : 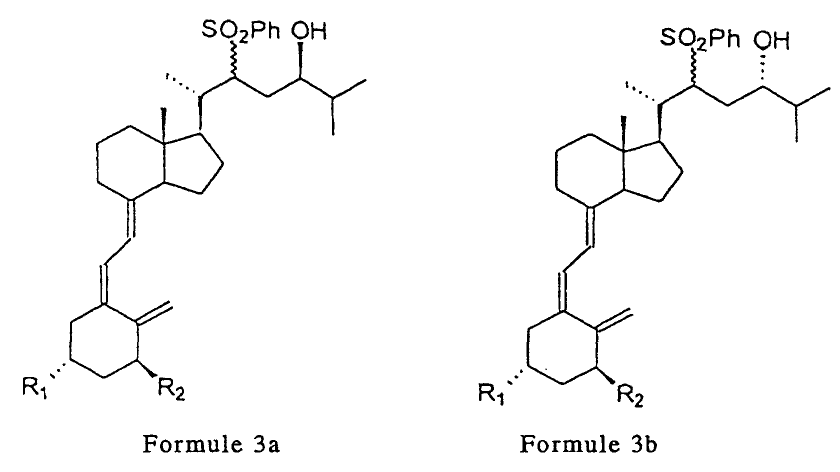 dans laquelle R₁ et R₂ ont les significations indiquées plus haut à propos de la formule 2,
ce qui donne un composé de formule 1, duquel on élimine éventuellement les groupes hydroxy-protecteurs, en opérant selon un procédé connu.

2. Procédé conforme à la revendication 1, dans lequel on emploie, en tant que base organique, un composé organique du lithium.

3. Procédé conforme à la revendication 1 ou 2, dans lequel on emploie, en tant que solvant aprotique, de l'hexaméthylphosphotriamide.

4. Procédé conforme à la revendication 1, 2 ou 3, dans lequel on emploie, en tant que composé réducteur, un amalgame de sodium.

5. Dérivé de cholécalciférol, qui est un composé de formule 3a : 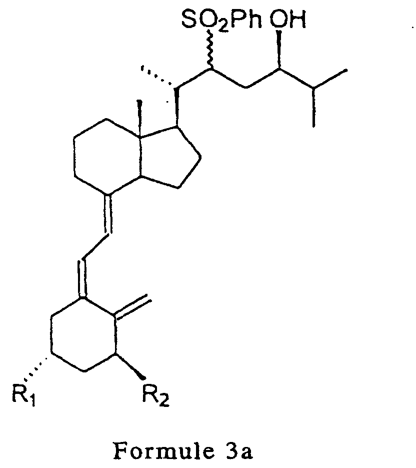 et de configuration représentée par l'une des formules 3c et 3d : 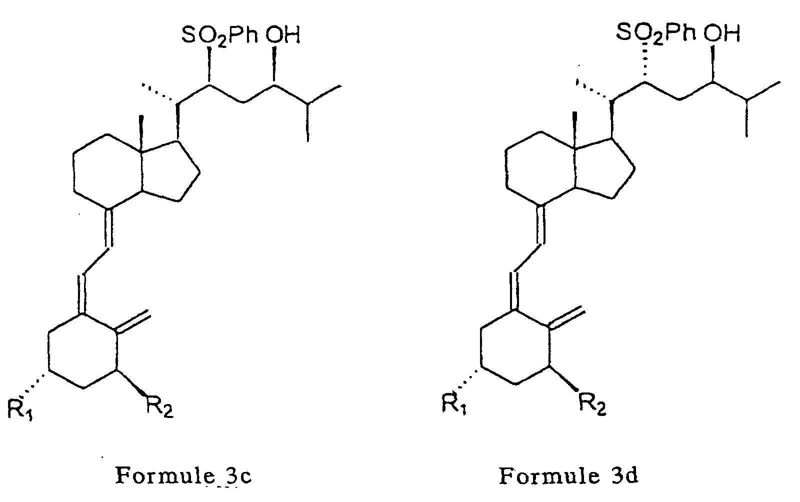 dans lesquelles R₁ et R₂ sont identiques ou différents et représentent chacun un atome d'hydrogène ou un groupe hydroxy-protecteur.

6. Dérivé de cholécalciférol, qui est un composé de formule 3b : 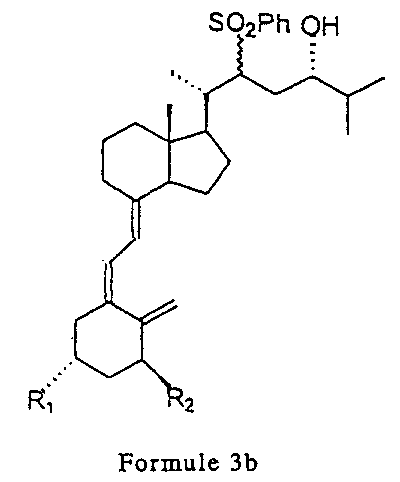 dans lesquelles R₁ et R₂ sont identiques ou différents et représentent chacun un atome d'hydrogène ou un groupe hydroxy-protecteur.

7. Dérivé de cholécalciférol, qui est un composé de formule 1 : 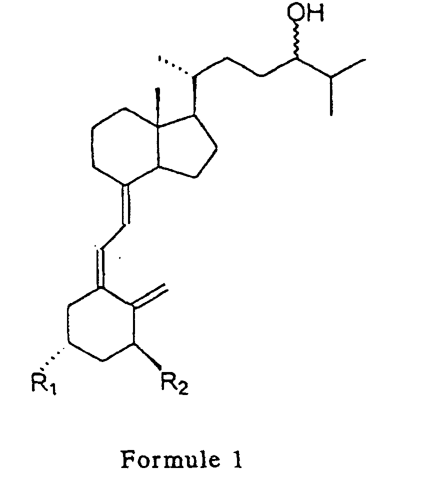 et de configuration représentée par l'une des formules 4a et 4b : 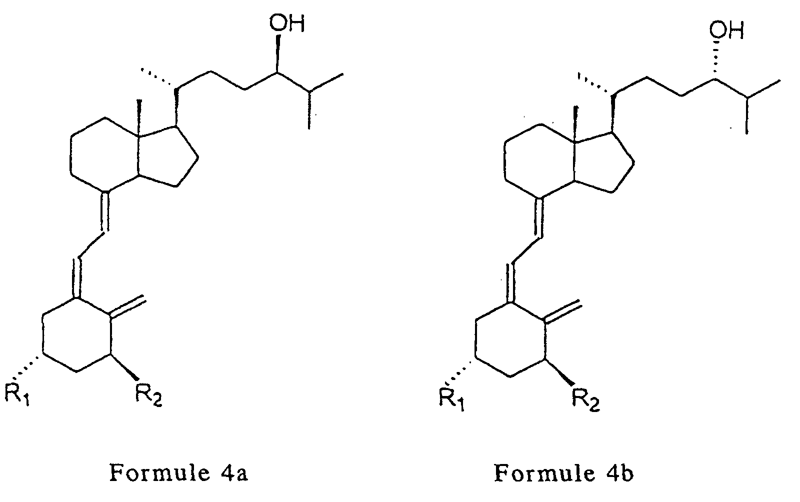 dans lesquelles R₁ et R₂ sont identiques ou différents et représentent chacun un atome d'hydrogène ou un groupe hydroxy-protecteur.
